# EUROPEAN PATENT SPECIFICATION

(11) **EP 2 636 410 B1**
(45) Date of publication and mention of the grant of the patent: **08.04.2015**
(21) Application number: 13001033.3
(22) Date of filing: 01.03.2013
(51) Int. Cl.: A61K 31/555, A61P 35/00

(54) **Dichlorido complexes of platinum with 7-azaindole halogeno-derivatives for use in the treatment of tumour diseases**
Dichlorkomplexe von Platin mit 7-Azaindol-Halogeno-Derivaten zur Verwendung bei der Behandlung von Tumorerkrankungen
Complexes dichlorido de platine avec des dérivatifs halogènes 7-azaindole et leur utilisation pour le traitement de maladies tumorales

(30) Priority: 07.03.2012 CZ 20120159
(43) Date of publication of application: 11.09.2013
(73) Proprietor: Univerzita Palackého, 771 47 Olomouc (CZ)
(72) Inventor: Trávnícek, Zdenek, 783 13 Stepánov (CZ); Starha, Pavel, 783 75 Dub nad Moravou (CZ); Dvorák, Zdenek, 779 00 Olomouc (CZ)
(74) Representative: Soukup, Petr

(56) References cited:
- CZ-A- 201 2-0 159
- CZ-U1- 23 204
- PAVEL STARHA ET AL: "How to modify 7-azaindole to form cytotoxic Pt(II) complexes: Highly in vitro anticancer effective cisplatin derivatives involving halogeno-substituted 7-azaindole", JOURNAL OF INORGANIC BIOCHEMISTRY, vol. 115, 1 October 2012 (2012-10-01), pages 57-63, XP055060692, ISSN: 0162-0134, DOI: 10.1016/j.jinorgbio.2012.05.006
- HARRISON R C ET AL: "In vivo properties of some new cis-platinum complexes containing 7-azaindole ligands", INORGANICA CHIMICA ACTA, ELSEVIER BV, NL, vol. 92, no. 1, 1 May 1984 (1984-05-01), pages 43-46, XP026606980, ISSN: 0020-1693, DOI: 10.1016/S0020-1693(00)80063-8 [retrieved on 1984-05-01]
- JOSÉ RUIZ ET AL: "New 7-azaindole palladium and platinum complexes: crystal structures and theoretical calculations. In vitro anticancer activity of the platinum compounds", DALTON TRANSACTIONS, vol. 39, no. 13, 1 April 2010 (2010-04-01) , pages 3290-3301, XP055060759, ISSN: 1477-9226, DOI: 10.1039/b920854b

## Description

### Field of the Invention

The invention relates to the utilization of the platinum(II) dichlorido complexes involving the 7-azaindole halogeno-derivatives for the preparation of drugs for the treatment of human tumour diseases, concretely for the treatment of ovarian carcinoma, prostate carcinoma, lung carcinoma, cervix carcinoma or malignant melanoma.

### Background of the Invention

*Cis*-diammine-dichloridoplatinum(II) complex, *cis*-[Pt(NH₃)₂C)₂], (*cisplatin*) is clinically used platinum(II) dichlorido complex. It is applied in the anticancer therapy and it is effective in the treatment of carcinomas (*e.g.* lung carcinoma or ovarian carcinoma), sarcomas or lymphomas. The therapeutic application of *cisplatin* is, however, connected with several negative side-effects, such as nephrotoxicity, neurotoxicity, myelosuppression or nausea. Moreover, many tumour types show intrinsic or acquired resistance to *cisplatin.* Due to the mentioned reasons, one of the directions of the research of antitumor active substances is the preparation of the platinum complexes, which show higher activity and their application is not accompanied with the resistance problems and the mentioned negative side-effects.

From the bioinorganic chemistry point of view, two ligand types can be distinguished within the *cisplatin* molecule, namely so called "*leaving ligands*" (i.e. two chloride anions, which are substituted under the physiological conditions by OH⁻ and/or H₂O, and the platinum(II) formed species are subsequently coordinated through these positions to the targeted nucleobases of the DNA molecule) and "*carrier ligands*" (i.e. two NH₃ molecules, which are not substituted under the physiological conditions). In accordance with the current state of scientific knowledge, it is apparent, that substitution of at least one of the ligand types or both ligand types will affect the resulting antitumor activity of the platinum(II) complexes.

The present platinum(II) dichlorido complexes with halogeno-derivatives of 7-azaindole (L), *cis*-[Pt(L)₂Cl₂], represent the *cisplatin* derivatives, which differ from the initial compound in the "*carrier ligands*"*,* since both NH₃ molecules are replaced by 7-azaindole halogeno-derivatives coordinated through their nitrogen N7 atoms. These recently prepared complexes were found to be substantially more *in vitro* antitumor active against human cancer cell lines of osteosarcoma HOS and breast adenocarcinoma MCF7 as compared to *cisplatin*, as it is reported in the utility model CZ 23204 U1 and national patent CZ 303417 B6, where the background of the invention is given in more details.

### Summary of the Invention

The invention provides the novel utilization of dichlorido complexes of platinum in the oxidation state +II and their crystal-solvates having the structural formula I, where at least one of the **R3, R4** and **R5** substituents is chosen from the group of: chlorine, bromine, or iodine, for the preparation of drugs for the treatment of ovarian carcinoma and/or prostate carcinoma and/or lung carcinoma and/or cervix carcinoma and/or malignant melanoma.

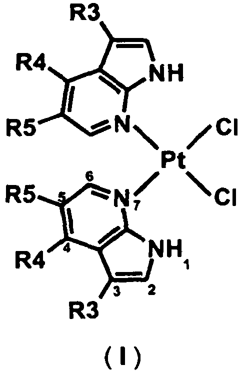

In continuity with the current research results, it was unexpectedly observed, that the platinum(II) dichlorido complex with halogeno-derivatives of 7-azaindole having the general formula *cis*-[PtCl₂(L)₂] and their crystal-solvates show significant *in vitro* antitumor activity against a wide spectrum of the human cancer cell lines, concretely ovarian carcinoma, prostate carcinoma, lung carcinoma, cervix carcinoma or malignant melanoma. The subsequent study on the mentioned human cancer cell lines and the obtained results proved that effect of these complexes is more broad-spectrum than it has been reported in CZ 23204 U1 and CZ 303417 B6, which enhance an applicability of the prepared compounds. It has to be pointed out, that the cause and carrier of the activity is the complex itself, and not the reaction components, which were found to be inactive on the tested tumours. A benefit of the invention is the fact that the complexes are significantly more active as compared with *cisplatin* in the most cases, e.g. more than ten-times against *cisplatin*-resistant ovarian carcinoma cell line (A2780R).

The broad-spectrum of antitumor activity, and thus the spectrum of the possible use of the present compounds, together with the selectivity of action against cisplatin-resistant ovarian carcinoma cell line (A2780R) represents the benefit of the invention to the current state of knowledge.

### Description of the figures

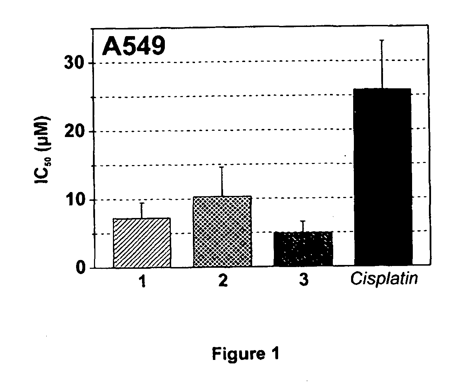

The concrete examples of the invention are documented by the attached drawings, where
- Fig. 1 is graph of the *in vitro* antitumor activity of the complexes *cis*-[PtCl₂(L₂)₂] (**1**), *cis*-[PtCl₂(L₃)₂]·0.75EtOH (**2**), *cis*-[PtCl₂(Le)₂] (**3**) against human cancer cell line of lung carcinoma A549 and their comparison with *cisplatin*; L₂ = 3-chloro-7-azaindole, L₃ = 3-iodo-7-azaindole, L₆ = 5-bromo-7-azaindole

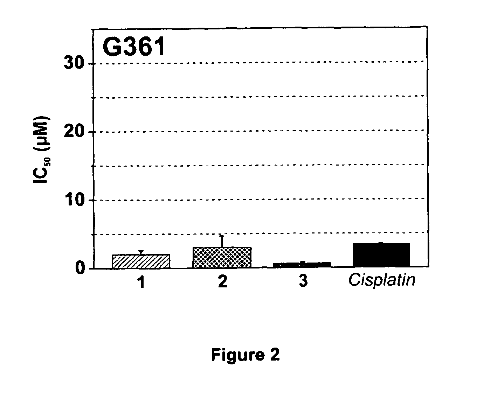

- Fig. 2 is graph of the *in vitro* antitumor activity of the complexes *cis*-[PtCl₂(L₂)₂] (**1**), *cis*-[PtCl₂(L₃)₂]·0.75EtOH (**2**), *cis*-[PtCl₂(L₆)₂] (**3**) against human cancer cell line of malignant melanoma G361 and their comparison with *cisplatin*; L₂ = 3-chloro-7-azaindole, L₃ = 3-iodo-7-azaindole, L₆ = 5-bromo-7-azaindole

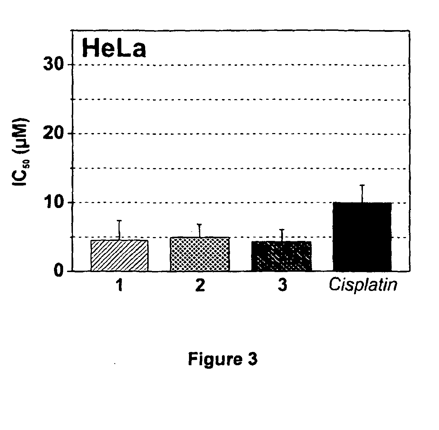

- Fig. 3 is graph of the *in vitro* antitumor activity of the complexes *cis*-[PtCl₂(L₂)₂] (**1**), *cis*-[PtCl₂(L₃)₂]·0.75EtOH (**2**), *cis*-[PtCl₂(L₆)₂] (**3**) against human cancer cell line of cervix carcinoma HeLa and their comparison with *cisplatin*; L₂ = 3-chloro-7-azaindole, L₃ = 3-iodo-7-azaindole, L₆ = 5-bromo-7-azaindole

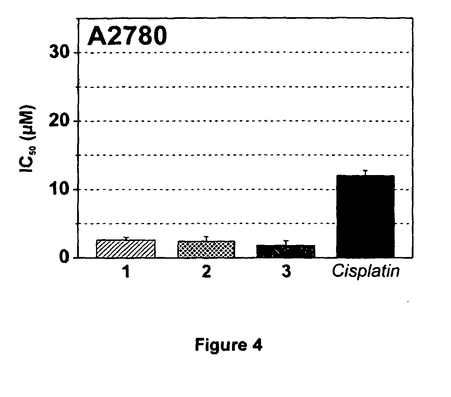

- Fig. 4 is graph of the *in vitro* antitumor activity of the complexes *cis*-[PtCl2₃(L₂)₂] (**1**), *cis*-[PtCl₂(L₃)₂]·0.75EtOH (**2**), *cis*-[PtCl₂(L₆)₂] (**3**) against human cancer cell line of ovarian carcinoma A2780 and their comparison with *cisplatin*; L₂ = 3-chloro-7-azaindole, L₃ = 3-iodo-7-azaindole, L₆ = 5-bromo-7-azaindole

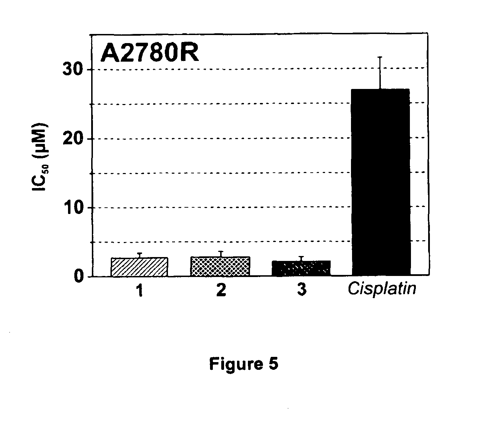

- Fig. 5 is graph of the *in vitro* antitumor activity of the complexes *cis*-[PtCl₂(L₂)₂] (**1**), *cis*-[PtCl₂(L₃)₂]·0.75EtOH (**2**), *cis*-[PtCl₂(L₆)₂] (**3**) against human cancer cell line of *cisplatin*-resistant ovarian carcinoma A2780R and their comparison with *cisplatin*; L₂ = 3-chloro-7-azaindole, L₃ = 3-iodo-7-azaindole, L₆ = 5-bromo-7-azaindole

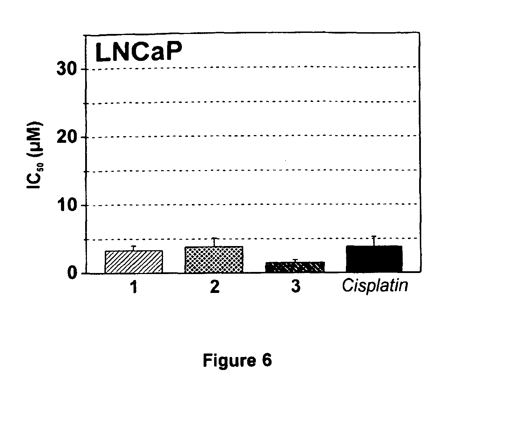

- Fig. 6 is graph of the *in vitro* antitumor activity of the complexes *cis*-tPtCl₂(L₂)₂] (**1**), *cis*-[PtCl₂(L₃)₂]·0.75EtOH (**2**), *cis*-[PtCl₂(L₆)₂] (**3**) against human cancer cell line of prostate carcinoma LNCaP and their comparison with *cisplatin*; L₂ = 3-chloro-7-azaindole, L₃ = 3-iodo-7-azaindole, L₆ = 5-bromo-7-azaindole

### Examples

In the following section, the invention is documented but not limited, by the concrete examples of its realization. The invention range is unambiguously limited by the invention claims.

The cytotoxic activities of the prepared complexes, as it is given below in the examples 1-6, were tested by micro-titration analysis using 3-(4,5-dimethylthiazol-2-yl)-2,5-diphenyltetrazolium bromide (MTT). The test detected living cells, whose number corresponds to the reduced MTT quantity. *In vitro* cytotoxicity was tested against the following human cancer cells: lung carcinoma A549, malignant melanoma G361, cervix carcinoma HeLa, ovarian carcinoma A2780, *cisplatin*-resistant ovarian carcinoma A2780R and prostate carcinoma LNCaP. The cells were maintained in plastic tissue culture flasks in the DMEM medium (5 g/L of glucose, 2 mM of glutamine, 100 U/mL of penicillin, 100 µg/mL of streptomycine, 10% fetal calf serum and sodium hydrogen carbonate) for cell cultures. The cell suspensions (*ca* 1.25 x 10⁵ cells mL⁻¹) were pipetted (80 µL) into 96-well microplates. They were incubated at 37 °C in the CO₂ atmosphere for 24 h. The tested dichlorido complexes of platinum were dissolved in *N,N'*-dimethylformamide (DMF) and diluted to the 50.0 mM concentration - these solutions represent the stock solutions. The stock solutions were diluted in DMF, giving the dilution series up to the 50.0 µM concentration. The dilution series of substances were diluted into culture medium. Medium with the studied complexes was added to each well with pre-incubated cell suspension. Then the mixtures were incubated for 24 h at 37 °C, 100% humidity and in the CO₂ atmosphere. The MTT solution was added followed by the incubation lasting 1 h. The MTT analysis was performed spectrofotometrically (TECAN, Schoeller Instruments LLC) at 540 nm.

The concentrations of the tested complexes (µM) lethal for 50% of the cancer cells (inhibition constant IC₅₀) were counted from the appropriate dose curves and the values are given in Table 1 a graphically depicted in Figure 4.

### Example 1: In vitro antitumor activity of the studied complexes against human cancer cell line of lung carcinoma A549 and their comparison with cisplatin

The *in vitro* antitumor activity of the studied complexes *cis*-[PtCl₂(L₂)₂] (**1**), *cis-*[PtCl₂(L₃)₂]·0,75EtOH (**2**) and *cis*-[PtCl₂(L₆)₂] (**3**) against human cancer cell line of lung carcinoma A549 was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 7.2±2.3 (complex **1**), 10.3±4.3 (complex **2**) and 4.9±1.7 (complex **3**), the value for *cisplatin* is 25.8±7.1 (Fig. 1).

### Example 2: In vitro antitumor activity of the studied complexes against human cancer cell line of malignant melanoma G361 and their comparison with cisplatin

The *in vitro* antitumor activity of the studied complexes *cis*-[PtCl₂(L₂)₂] (**1**), *cis-*[PtCl₂(L₃)₂]·0,75EtOH (**2**) and *cis*-[PtCl₂(L₆)₂] (**3**) against human cancer cell line of malignant melanoma G361 was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 2.0±0.6 (complex **1**), 3.0±1.7 (complex **2**) and 0.6±0.2 (complex **3**), the value for *cisplatin* is 3.4±0.1 (Fig. 2).

### Example 3: In vitro antitumor activity of the studied complexes against human cancer cell line of cervix carcinoma HeLa and their comparison with cisplatin

The *in vitro* antitumor activity of the studied complexes *cis*-[PtCl₂(L₂)₂] (**1**), *cis-*[PtCl₂(L₃)₂]·0,75EtOH (**2**) and *cis*-[PtCl₂(L₆)₂] (**3**) against human cancer cell line of cervix carcinoma HeLa was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 4.5±2.9 (complex **1**), 5.0±1.9 (complex **2**) and 4.3±1.8 (complex **3**), the value for *cisplatin* is 10.0±2.6 (Fig. 3).

### Example 4: In vitro antitumor activity of the studied complexes against human cancer cell line of ovarian carcinoma A2780 and their comparison with cisplatin

The *in vitro* antitumor activity of the studied complexes *cis*-[PtCl₂(L₂)₂] (**1**), *cis-*[PtCl₂(L₃)₂]·0,75EtOH (**2**) and *cis*-[PtCl₂(L₆)₂] (**3**) against human cancer cell line of ovarian carcinoma A2780 was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 2.6±0.4 (complex **1**), 2.4±0.7 (complex **2**) and 1.8±0.7 (complex **3**), the value for *cisplatin* is 12.0±0.8 (Fig. **4**).

### Example 5: In vitro antitumor activity of the studied complexes against human cancer cell line of cisplatin-resistant ovarian carcinoma A2780R and their comparison with cisplatin

The *in vitro* antitumor activity of the studied complexes *cis*-[PtCl₂(L₂)₂] (**1**), cis-[PtCl₂(L₃)₂]·0.75EtOH (**2**) and *cis*-[PtCl₂(L₆)₂] (**3**) against human cancer cell line of *cisplatin*-resistant ovarian carcinoma A2780R was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 2.7±0.7 (complex **1**), 2.8±0.8 (complex **2**) and 2.1±0.7 (complex **3**), the value for *cisplatin* is 27.0±4.6 (Fig. 5).

### Example 6: In vitro antitumor activity of the studied complexes against human cancer cell line of prostate carcinoma LNCaP and their comparison with cisplatin

The *in vitro* antitumor activity of the studied complexes *cis*-[PtCl₂(L₂)₂] (**1**), *cis-*[PtCl₂(L₃)₂]·0.75EtOH (**2**) and *cis*-[PtCl₂(L₆)₂] (**3**) against human cancer cell line of prostate carcinoma LNCaP was determined by the above-described micro-titration analysis using MTT (L₂ = 3-chloro-7-azaindole, L₃ = 3-iodo-7-azaindole, L₆ = 5-bromo-7-azaindole).

The inhibition concentrations of the studied complexes IC₅₀ (µM) equal 3.3±0.7 (complex **1**), 3.8±1.3 (complex **2**) and 1.5±0.4 (complex **3**), the value for *cisplatin* is 3.8±1.5 (Fig. 6).

## Claims

1. Dichlorido complexes of platinum in the oxidation state +11 and their crystal-solvates having the structural formula (I), 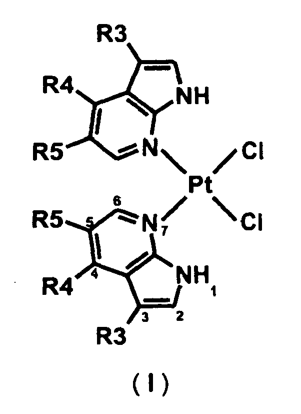 where at least one of the hydrogen atoms localized on the carbon atoms C3, C4 or C5 of the 7-azaindole skeleton is substituted at least one atom from the group of: chlorine, bromine, or iodine, for use in the treatment of ovarian carcinoma and/or prostate carcinoma and/or lung carcinoma and/or cervix carcinoma and/or malignant melanoma.

## Patentansprüche

1. Die Dichloridkomplexen des Platins in dem Oxidationszustand +II und ihre Kristallsolvaten haben die strukturelle Formel (I), 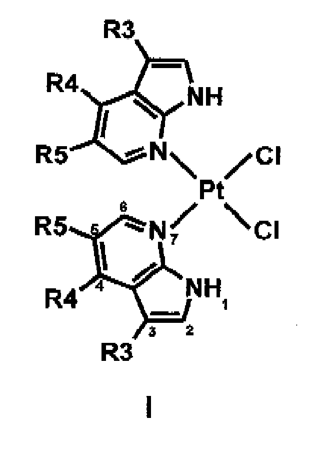 wo mindestens eine von Hydrogensatome auf dem Karbonsatome C3, C4 oder C5 von 7-azaindole Skeleton platziert ist, und ersetzte mit mindestens ein Atom von der Gruppe: das Chlor, das Brom oder das Jod, mit dem Gebrauch in die Heilung von Eierstockkrebs und/oder Vorsteherdrüsekrebs und/oder Lungenkrebs und/oder Gebärmutterzäpfchen und/oder malignen Melanom.

## Revendications

1. Les complexes dichlores de platine au degré d'oxydation +II et leurs solvates cristals sur la base de la formule structurelle (I), 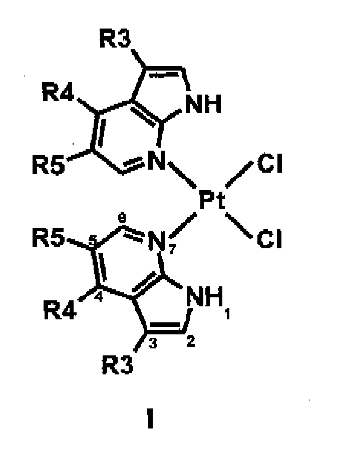 quand au moins l'un des atomes d'hydrogène situés sur les atomes de carbone C3, C4 ou C5 dans le squelette 7- azaindole est substiué ou remplacé par au moins un atome du groupe du chlore, du brome ou de l'iode pour l'utilisation dans le traitement du cancer de l'ovaire et/ou le cancer de la prostate et/ou le cancer du poumon et/ou le cancer du col utérin et/ou du mélanome malin.
